# FASCICULE DE BREVET EUROPEEN

(11) **EP 2 855 444 B1**
(45) Date de publication et mention de la délivrance du brevet: **20.07.2016**
(21) Numéro de dépôt: 13723875.4
(22) Date de dépôt: 25.04.2013
(51) Int. Cl.: C07D 401/04, H01M 10/052

(54) **SEL D'ANIONS BICYCLIQUES AROMATIQUES POUR BATTERIES LI-ION**
SALZ AUS BICYCLISCHEN AROMATISCHEN ANIONEN FÜR LI-IONEN-BATTERIEN
SALT OF BICYCLIC AROMATIC ANIONS FOR LI-ION BATTERIES

(30) Priorité: 04.06.2012 FR 1255152
(43) Date de publication de la demande: 08.04.2015
(73) Titulaire: ARKEMA FRANCE, 92705 Colombes Cedex (FR)
(72) Inventeur: SCHMIDT, Grégory, 69700 Saint Andéol le Château (FR)
(74) Mandataire: Dang, Doris
(86) Numéro de dépôt international: PCT/FR2013/050926
(87) Numéro de publication internationale: WO 2013/182767

(56) Documents cités:
- WO-A1-2010/023413
- US-A1- 2004 009 393

## Description

### DOMAINE DE L'INVENTION

La présente invention concerne les composés imidazole bicycliques, et leurs sels, leurs procédés de fabrication ainsi que leurs utilisations, notamment comme composant d'électrolyte pour batteries.

### ARRIERE-PLAN TECHNIQUE

Une batterie lithium-ion ou sodium-ion comprend au moins une électrode négative, une électrode positive, un séparateur et un électrolyte. L'électrolyte est constitué d'un sel de lithium ou sodium dissous dans un solvant qui est généralement un mélange de carbonates organiques, afin d'avoir un bon compromis entre la viscosité et la constante diélectrique.

Parmi les sels les plus utilisés figure l'hexafluorophosphate de lithium (LiPF6), qui possède beaucoup des nombreuses qualités requises mais présente le désavantage de se dégrader sous forme de gaz d'acide fluorhydrique. Cela pose des problèmes de sécurité, notamment dans le contexte de l'utilisation prochaine des batteries lithium-ion pour les véhicules particuliers.

Les prérequis pour avoir un sel d'électrolyte sont une bonne dissociation chimique entre le cation et l'anion ce qui implique une charge négative sur l'anion délocalisée ou diminuée par des effets attracteurs.

Des sels basés sur l'effet attracteur ont donc été développés tels que le LiTFSI (bis(trifluoromethanesulfonyl)imidure de lithium) et le LiFSI (bis(fluorosulfonyl)imidure de lithium).

D'autres sels cette fois basés sur la délocalisation de la charge ont également été developpés tels que le LiTDI (1-trifluorométhyl-4,5-dicarbonitrile-imidazolate de lithium), ainsi que cela est enseigné dans le document WO 2010/023413. Mais ces derniers présentent des conductivités ioniques plus faibles que ceux précédemment cités.

La demanderesse a découvert que la présence d'un second cycle aromatique permettrait d'augmenter la délocalisation de la charge négative et ainsi augmenter cette conductivité ionique.

### RESUME DE L'INVENTION

Dans ce qui suit, on désigne par
- DAMN : le diaminomaléonitrile et est représenté par la formule (I) 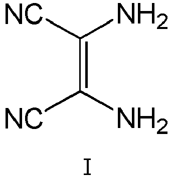
- Les composés (II) sont représentés par les formules générales développées ci-dessous. Ils sont désignés sous (IIa) lorsque le cycle aromatique est à 6 atomes et sous (IIb) pour un cycle aromatique à 5 atomes 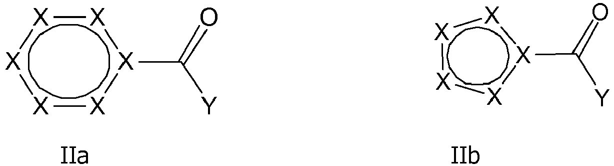
- Les composés imidazole bicycliques (III) sont représentés par les formules générales développées ci-dessous. Ils sont désignés sous (IIIa) lorsque le cycle aromatique est à 6 atomes et sous (IIIb) pour un cycle aromatique à 5 atomes 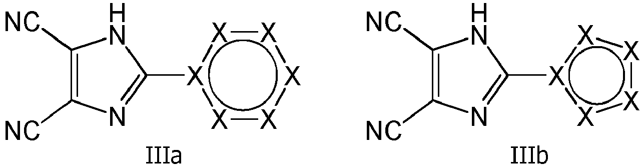
- Les sels des composés imidazole bicycliques (IV) sont représentés par les formules générales développées ci-dessous. Ils sont désignés sous (IVa) lorsque le cycle aromatique est à 6 atomes et sous (IVb) pour un cycle aromatique à 5 atomes 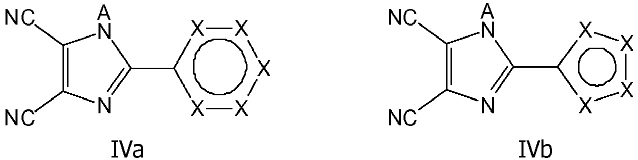
- Les composés (V) sont représentés par les formules générales développées ci-dessous. Ils sont désignés sous (Va) lorsque le cycle aromatique est à 6 atomes et sous (Vb) pour un cycle aromatique à 5 atomes

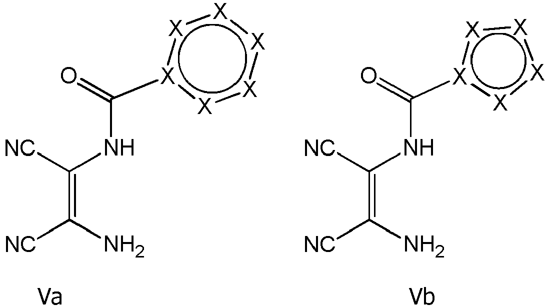

Dans les formules générales ci-dessus, A représente un cation monovalent, X représente indépendamment un atome de carbone, un atome d'oxygène, un atome de soufre, un atome de phosphore ou un atome d'azote et Y représente un atome de chlore, un groupement OCORf dans lequel Rf représente le même cycle aromatique que dans les composés IIa et IIb ou un groupement OR', dans lequel R' représente un groupement alkyle de 1 à 5 atomes de carbone éventuellement substitué par un ou plusieurs atomes de fluor.

Lorsque X représente un atome de carbone ou phosphore ou azote, les substituants peuvent être indépendamment des groupements électro-attracteurs ou électro-donneurs définis par un paramètre de Hammett (le paramètre de Hammett est une constante tabulée qui est déterminée pour une série de groupes substituants en mesurant la constante de dissociation des acides benzoïques correspondants) compris entre -0,7 et 1,0. De préférence, les substituants sont choisis parmi comme un groupement cyano (CN), un groupement R₁, un groupement éther de type OR₁, un groupement amino de type N(R₁)₂, un groupement ester de type CO₂R₁, un groupement sulfonyle type SO₂R₁ ou un groupement phosphonyle type PO₂R₁, où R₁ a pour formule CₙHₘX'ₚ avec n compris entre 0 et 6, m compris entre 0 et 13, X' est un halogène (F, CI, Br et I) et p compris entre 1 et 13.

Lorsque Y représente un atome de chlore, un groupement OCORf ou OR', le composé (II) est respectivement un chlorure d'acyle, un anhydride symétrique ou un ester.

L'invention concerne en premier lieu les composés imidazole bicycliques (III) et leurs sels (IV).

L'invention concerne en deuxième lieu les procédés de fabrication de composés imidazole bicycliques (III) et leurs sels (IV).

L'invention concerne en troisième lieu l'utilisation des composés de formule (IV).

### DESCRIPTION DETAILLEE DE L'INVENTION

L'invention est maintenant décrite plus en détail et de façon non limitative dans la description qui suit.

Les sels des composés imidazole bicycliques (IV), selon la présente invention sont représentés par la formule générale ci-dessus dans laquelle A représente un cation monovalent A, par exemple un métal alcalin.

Le métal alcalin préféré est choisi parmi le lithium et le sodium.

Lorsque X dans la formule générale représente un atome de carbone, de phosphore ou d'azote, les sels (IV) peuvent être substitués. Les substituants préférés sont des groupements électro-attracteurs ou électro-donneurs, en particulier ceux ayant un paramètre de Hammett compris entre -0,7 et 1.

Les groupements électro-attracteurs et électro-donneurs particulièrement préférés peuvent être choisis parmi un groupement cyano (CN), un groupement R₁, un groupement éther de type OR₁, un groupement amino de type N(R₁)₂, un groupement ester de type CO₂R₁, un groupement sulfonyle type SO₂R₁ ou un groupement phosphonyle type PO₂R₁, où R₁ a pour formule CₙHₘX'ₚ avec n compris entre 0 et 6, m compris entre 0 et 13, X' est un halogéne (F, CI, Br et I) et p compris entre 1 et 13.

### Préparation des sels des composés d'imidazole bicycliques (imidazolates bicycliques) et des composés d'imidazole bicycliques

Les imidazolates bicycliques (IV) peuvent être préparés à partir des composés imidazole (III) en faisant réagir celui-ci avec une base AZ, avec A ayant la même signification que ci-dessus et Z représentant un anion hydrure, hydroxyde ou carbonate. De préférence AZ est choisi parmi l'hydrure de lithium, le carbonate de lithium, l'hydroxyde de lithium, l'hydrure de sodium, le carbonate de sodium, l'hydroxyde de sodium et les combinaisons de ceux-ci.

III + AZ → IV + AH (1)

Les composés (III) peuvent être préparés à partir du DAMN et d'un dérivé d'acide cyclique aromatique de formule générale (II).

Le procédé de préparation des composés imidazole bicycliques (III) comprend (i) une étape réactionnelle du DAMN de formule (I) avec un dérivé d'acide cyclique aromatique de formule (II) à une température T₁ comprise entre_0 et 80°C, de préférence de 10 à 50°C, plus préférentiellement de 20 à 30°C, en présence éventuellement d'un solvant pour donner un composé de formule (V), suivie (ii) d'une étape au cours de laquelle le composé de formule (V) est soumis à un traitement thermique à une température T₂ avec T₂ > T₁.

DAMN + II → III+H₂O+YH (2)

DAMN + II → V + YH (2-1)

V → III + H₂O (2-2)

De préférence, T₂ est supérieure à T₁ d'au moins 10°C, ou d'au moins 20°C, ou d'au moins 30°C, ou d'au moins 40°C, ou d'au moins 50°C, ou d'au moins 60°C, ou d'au moins 70°C.

De préférence, l'étape (ii) est effectuée immédiatement à la suite de la première étape sans purification intermédiaire, simplement en modifiant la température du mélange réactionnel, par chauffage éventuellement après une période de transition, de préférence comprise entre 1 minute et 2 heures.

L'étape (i) est de préférence mise en oeuvre en présence d'un solvant. Tout composé permettant de solubiliser le(s) réactif(s) peut être utilisé comme solvant. A titre indicatif, on peut citer le dioxane, le toluène, l'acétonitrile ou le diméthylformamide.

Lorsque l'étape (i) est mise en oeuvre en présence d'un solvant, la concentration du DAMN dans le milieu réactionnel est de préférence de 0,001 à 2 mol/l, plus préférentiellement de 0,1 mol/l à 1 mol/l. Le rapport molaire du composé (I) sur le composé (II) est de préférence de 0,25 à 1,5, plus préférentiellement de 0,5 à 1,25.

Selon un mode de réalisation particulier, la température T₂ correspond à la température d'ébullition du solvant utilisé.

La durée de l'étape (i) est de préférence de 1 à 12 heures, plus particulièrement de 1 à 5 heures, par exemple d'environ 2 heures.

De préférence, l'étape (ii) est mise en oeuvre en présence d'un catalyseur acide, éventuellement par ajout d'un acide carboxylique, tel que l'acide trifluoroacétique, l'acide acétique ou l'acide benzoïque dans le milieu réactionnel.

Le catalyseur acide peut être obtenu in-situ dans le milieu réactionnel, notamment lorsque le composé de formule (II) est un anhydride.

Le rapport molaire du composé (V) sur le catalyseur est de préférence de 0,5 à 20, plus préférentiellement de 1 à 10.

Selon un mode de réalisation de l'invention, la température de la réaction T₁ peut être constante tout au long de la première étape, et/ou la température de la réaction T₂ peut être constante tout au long de la deuxième étape.

Selon un autre mode de réalisation de l'invention, la température est croissante tout au long de l'étape (i) et éventuellement tout le long de l'étape (ii) à la condition que T₂ est supérieure à T₁. Autrement dit, la température minimale T₂ est supérieure à la température maximale T₁.

A l'issue de l'étape (ii), le composé imidazole bicyclique de formule (III) est de préférence isolé et purifié.

Ainsi, le milieu réactionnel peut être évaporé et l'imidazole (III) recristallisé dans de l'eau pour ensuite être récupéré par filtration. Le solide obtenu peut être dissous dans une solution aqueuse de base AZ, de préférence lithiée ou sodée avec une concentration allant de 10⁻⁵ mol/l à la concentration de saturation. Une fois le sel de composé de formule (IV) est formé la solution peut subir plusieurs traitements au charbon actif. La solution peut ensuite être évaporée pour donner le sel de formule (IV).

### Préparation d'un électrolyte

Les composés de formule (IV) peuvent être utilisés pour la préparation d'un électrolyte, en les dissolvant dans un solvant approprié.

Le solvant peut être constitué d'au moins un composé choisi parmi les carbonates, les glymes, les nitriles et les sulfones.

Comme carbonate, on peut citer notamment l'éthylene carbonate, le carbonate de glycérol, le dimethylcarbonate, l'éthylméthylcarbonate, le diéthylcarbonate, le propylene carbonate.

Comme glymes on peut citer notamment l'éthylène glycol diméthyléther, le diéthylène glycol diméthyléther, le dipropylène glycol diméthyléther, le diéthylène glycol diéthyléther, le triéthylène glycol diméthyléther, le diéthylène glycol dibutyléther, le tétraéthylène glycol diméthyléther et le diéthylène glycol t-buthylméthyléther.

Comme nitriles on peut citer notamment, l'acétonitrile, le propionitrile, le butyronitrile, le méthoxypropionitrile, l'isobutyronitrile et les composés fluorés dérivants des composés précédents.

Comme sulfones on peut notamment citer le diméthylsulfone, le sulfolane, l'éthylméthylsulfone, le propylméthylsulfone, l'isopropylméthylsulfone, l'isopropyléthylsulfone, le tertbutyléthylsulfone, le tertbutylméthylsulfone et le tertbutylpropylsulfone.

Le solvant est de préférence constitué d'un mélange de composés, avantageusement de 2 à 5 choisis parmi les carbonates et/ou glymes et/ou les sulfones précédemment cités.

Les proportions en poids de chacun des composés constituant le solvant sont de préférence comprises entre 1 et 10 par rapport au constituant en plus faible quantité, plus préférentiellement entre 1 et 8.

La concentration en composé de formule (IV) dans l'électrolyte est de préférence de 0,1 mol/l à 5 mol/l, plus préférentiellement de 0,2 mol/l à 2,5 mol/l. De préférence, l'électrolyte est constitué d'un mélange d'au moins deux sels de lithium choisi parmi le sel d'imidazolate (IV), le LiPF₆, le LiBF₄, le CF₃COOLi, le CF₃SO₂Li, le LiTFSI (bis(trifluoromethanesulfonyl)imidure de lithium), le LiFSI (bis(fluorosulfonyl)imidure de lithium), le LiTDI (1-trifluorométhyl-4,5-dicarbonitrile-imidazolate de lithium) et le LiPDI (1-pentafluoroéthyl-4,5-dicarbonitrile-imidazolate de lithium). La quantité de chaque sel de lithium présent dans le mélange peut varier dans de larges limites et représente en général, entre 0,1 et 99,9 % en poids par rapport au poids total des sels présents dans le mélange, et de préférence entre 1 et 99 % en poids.

### EXEMPLE

L'exemple suivant illustre l'invention sans la limiter.

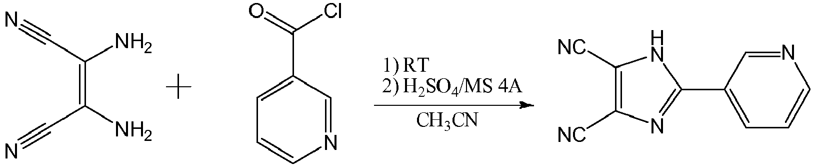

On ajoute goutte à goutte une solution de 1,82 g de chlorure de nicotinoyle dans 25 ml d'acétonitrile à une solution de 1,32 g de DAMN dans 20 ml d'acétonitrile. Un précité saumon apparaît alors. On ajoute ensuite le tamis moléculaire 4A et quelques gouttes d'acide sulfurique. Puis on chauffe le mélange à reflux pendant 36 heures. Le milieu réactionnel est filtré (élimination du tamis moléculaire) et est ensuite évaporé. L'imidazole est alors recristallisé dans de l'eau, il est ensuite récupéré par filtration. Le solide obtenu est dissous dans une solution aqueuse saturée en carbonate de lithium. Une fois le sel de formé, la solution subit 5 traitements de charbon actif (5 g). La solution est ensuite évaporée pour donner le sel de lithium de l'imidazolate.

## Revendications

1. Sels des composés imidazole bicycliques (IV) représentés par les formules générales développées ci-dessous : 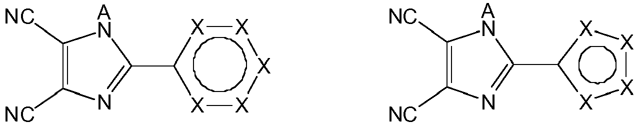 dans lesquelles A représente un cation monovalent, X représente indépendamment un atome de carbone, un atome d'oxygène, un atome de soufre, un atome de phosphore ou un atome d'azote.

2. Sels selon la revendication 1 **caractérisés en ce que** le cation monovalent A est un métal alcalin, de préférence choisi parmi le lithium ou le sodium.

3. Sels selon la revendication 1 ou 2 **caractérisés en ce que** X représente un atome de carbone, de phosphore ou azote.

4. Sels selon la revendication 3 **caractérisés en ce que** lesdits sels sont substitués par un groupement électro-attracteur ou électro-donneur choisi parmi un groupement cyano (CN), un groupement R₁, un groupement éther de type OR₁, un groupement amino de type N(R₁)₂, un groupement ester de type CO₂R₁, un groupement sulfonyle type SO₂R₁ ou un groupement phosphonyle type PO₂R₁, où R₁ a pour formule CₙHₘX'ₚ avec n compris entre 0 et 6, m compris entre 0 et 13, X' est un halogène (F, CI, Br et I) et p compris entre 1 et 13.

5. Sels selon la revendication 4 **caractérisés en ce que** le groupement électro-attracteur ou électro-donneur est choisi parmi l'hydrogène, le fluor, le groupement cyano (CN), le groupe trifluorométhyle (CF₃), le groupe trifluorométhoxy (OCF₃) ou le groupe méthoxy (OCH₃).

6. Procédé de préparation des sels selon l'une quelconque des revendications 1 à 5 **caractérisé en ce que** l'on fait réagir des composés imidazole (III) représentés par les formules générales développées ci-dessous : 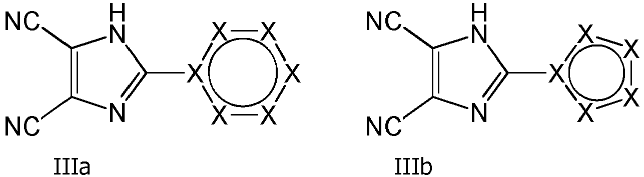 avec une base AZ, avec A ayant la même signification que ci-dessus et Z représentant un anion hydrure, hydroxyde ou carbonate.

7. Procédé selon la revendication 6 **caractérisé en ce que** la base AZ est choisi parmi l'hydrure de lithium, le carbonate de lithium, l'hydroxyde de lithium, l'hydrure de sodium, le carbonate de sodium, l'hydroxyde de sodium et les combinaisons de ceux-ci.

8. Procédé selon la revendication 6 ou 7 **caractérisé en ce que** les composés imidazole (III) sont obtenus en faisant réagir le diaminomaléonitrile avec un dérivé dérivé d'acide cyclique aromatique de formule (II) représenté par les formules générales développées ci-dessous :

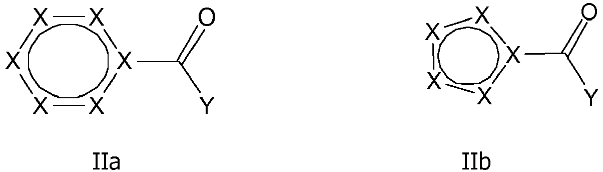

9. Procédé selon la revendication 8 comprenant (i) une étape réactionnelle du diaminomaléonitrile avec un dérivé d'acide cyclique aromatique de formule (II) à une température T₁ compris entre 0 et 80°C, de préférence de 10 à 50°C, plus préférentiellement de 20 à 30°C, en présence éventuellement d'un solvant pour donner un composé de formule (V) représenté par les formules générales développées ci-dessous : 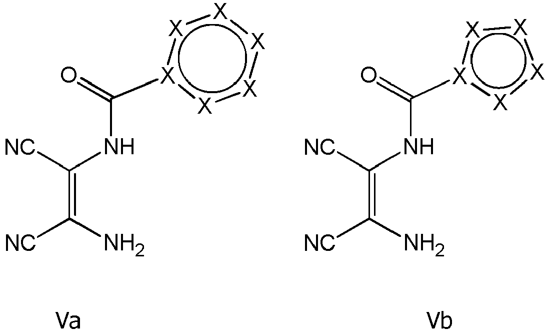 suivie (ii) d'une étape au cours de laquelle le composé de formule (V) est soumis à un traitement thermique à une température T₂ avec T₂ > T₁.

10. Procédé selon la revendication 9 **caractérisé en ce que** la température T₂ est supérieure à T₁ d'au moins 10°C, ou d'au moins 20°C, ou d'au moins 30°C, ou d'au moins 40°C, ou d'au moins 50°C, ou d'au moins 60°C, ou d'au moins 70°C.

11. Procédé selon la revendication 9 ou 10 **caractérisé en ce que** l'étape (ii) est effectuée immédiatement à la suite de la première étape sans purification intermédiaire.

12. Procédé selon l'une quelconque des revendications 8 à 11 **caractérisé en ce que** l'étape (i) est mise en oeuvre en présence d'un solvant.

13. Procédé selon la revendication 12 **caractérisé en ce que** le solvant est choisi parmi le dioxane, le toluène, l'acétonitrile ou le diméthylformamide.

14. Procédé selon l'une quelconque des revendications 8 à 13 **caractérisé en ce que** l'étape (ii) est mise en oeuvre en présence d'un catalyseur acide.

15. Procédé selon la revendication 14 **caractérisé en ce que** le catalyseur acide est choisi parmi l'acide trifluoroacétique, l'acide acétique ou l'acide benzoïque.

16. Procédé selon l'une quelconque des revendications 12 à 16 **caractérisé en ce que** la température T₂ correspond à la température d'ébullition du solvant.

17. Utilisation des sels selon l'une quelconque des revendications 1 à 5 comme composant électrolyte pour batterie.

18. Composition électrolyte comprenant outre les sels selon l'une quelconque des revendications 1 à 5, au moins un sel choisi parmi le LiPF₆, le LiBF₄, le CF₃COOLi, le CF₃SO₂Li, le LiTFSI (bis(trifluoromethanesulfonyl)imidure de lithium), le LiFSI (bis(fluorosulfonyl)imidure de lithium), le LiTDI (1-trifluorométhyl-4,5-dicarbonitrile-imidazolate de lithium) et le LiPDI (1-pentafluoroéthyl-4,5-dicarbonitrile-imidazolate de lithium).

19. Utilisation selon la revendication 17 **caractérisé en ce que** les sels sont solubilisés dans un solvant, de préférence choisi parmi les carbonates, les nitriles ou les glymes.

## Patentansprüche

1. Salze bicyclischer Imidazolverbindungen (IV) der folgenden allgemeinen Strukturformeln: 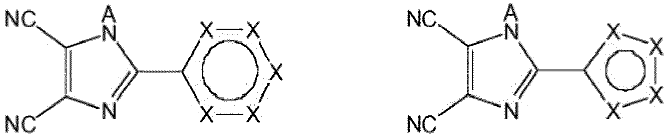 worin A ein einwertiges Kation bedeutet und die Atome X unabhängig voneinander ein Kohlenstoffatom, ein Sauerstoffatom, ein Schwefelatom, ein Phosphoratom oder ein Stickstoffatom bedeuten.

2. Salze nach Anspruch 1, **dadurch gekennzeichnet, dass** das einwertige Kation A ein Alkalimetall ist, das vorzugsweise unter Lithium oder Natrium ausgewählt ist.

3. Salze nach Anspruch 1 oder 2, **dadurch gekennzeichnet, dass** X ein Kohlenstoffatom, ein Phosphoratom oder ein Stickstoffatom bedeutet.

4. Salze nach Anspruch 3, **dadurch gekennzeichnet, dass** die Salze mit einer elektronenanziehenden oder elektronenliefernden Gruppe substituiert sind, die unter einer Cyanogruppe (CN), einer Gruppe R₁, einer Ethergruppe vom Typ OR₁, einer Aminogruppe vom Typ N(R₁)₂, einer Estergruppe vom Typ CO₂R₁, einer Sulfonylgruppe vom Typ SO₂R₁ oder einer Phosphonylgruppe vom Typ PO₂R₁ ausgewählt ist, wobei R₁ die Formel CₙHₘX'ₚ aufweist, worin n zwischen 0 und 6 liegt, m zwischen 0 und 13 liegt, X' ein Halogen (F, Cl, Br und I) bedeutet und p zwischen 1 und 13 liegt.

5. Salze nach Anspruch 4, **dadurch gekennzeichnet, dass** die elektronenanziehende oder elektronenliefernde Gruppe unter Wasserstoff, Fluor, der Cyanogruppe (CN), der Trifluormethylgruppe (CF₃), der Trifluormethoxygruppe (OCF₃) oder der Methoxygruppe (OCH₃) ausgewählt ist.

6. Verfahren zur Herstellung von Salzen nach einem der Ansprüche 1 bis 5, **dadurch gekennzeichnet, dass** die Imidazolverbindungen (III) gemäß den folgenden allgemeinen Strukturformeln: 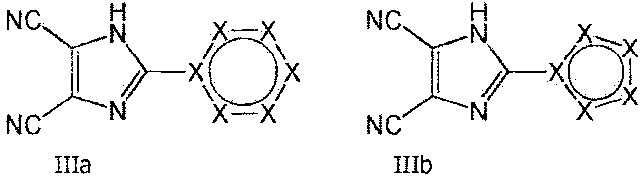 mit einer Base AZ umgesetzt werden, wobei A die oben angegebene Bedeutung hat und Z ein Hydrid-Anion, Hydroxid oder Carbonat bedeutet.

7. Verfahren nach Anspruch 6, **dadurch gekennzeichnet, dass** die Base AZ unter Lithiumhydrid, Lithiumcarbonat, Lithiumhydroxid, Natriumhydrid, Natriumcarbonat, Natriumhydroxid und deren Kombinationen ausgewählt ist.

8. Verfahren nach Anspruch 6 oder 7, **dadurch gekennzeichnet, dass** die Imidazolverbindungen (III) hergestellt werden, indem Diaminomaleonitril mit einem Derivat einer cyclischen aromatischen Säure der Formel (II) gemäß den folgenden allgemeinen Strukturformeln umgesetzt wird:

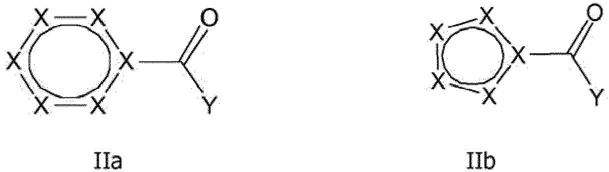

9. Verfahren nach Anspruch 8, das (i) einen Schritt der Umsetzung des Diaminomaleonitrils mit einem Derivat einer cyclischen aromatischen Säure der Formel (II) bei einer Temperatur T₁ zwischen 0 und 80 °C, vorzugsweise von 10 bis 50 °C und besonders bevorzugt von 20 bis 30 °C gegebenenfalls in Gegenwart eines Lösungsmittels zur Bildung einer Verbindung der Formel (V) gemäß den folgenden allgemeinen Strukturformeln: 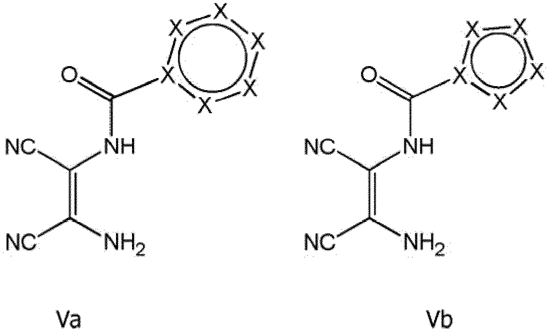 und anschließend (ii) einen Schritt umfasst, bei dem die Verbindung der Formel (V) einer thermischen Behandlung bei einer Temperatur T₂ mit T₂ > T₁ unterzogen wird.

10. Verfahren nach Anspruch 9, **dadurch gekennzeichnet, dass** die Temperatur T₂ mindestens 10 °C oder mindestens 20 °C oder mindestens 30 °C oder mindestens 40 °C oder mindestens 50 °C oder mindestens 60 °C oder mindestens 70 °C über T₁ liegt.

11. Verfahren nach Anspruch 9 oder 10, **dadurch gekennzeichnet, dass** Schritt (ii) ohne zwischenzeitliche Reinigung unmittelbar nach dem ersten Schritt durchgeführt wird.

12. Verfahren nach einem der Ansprüche 8 bis 11, **dadurch gekennzeichnet, dass** Schritt (i) in Gegenwart eines Lösungsmittels durchgeführt wird.

13. Verfahren nach Anspruch 12, **dadurch gekennzeichnet, dass** das Lösungsmittel unter Dioxan, Toluol, Acetonitril oder Dimethylformamid ausgewählt ist.

14. Verfahren nach einem der Ansprüche 8 bis 13, **dadurch gekennzeichnet, dass** Schritt (ii) in Gegenwart eines Säurekatalysators durchgeführt wird.

15. Verfahren nach Anspruch 14, **dadurch gekennzeichnet, dass** der Säurekatalysator unter Trifluoressigsäure, Essigsäure oder Benzoesäure ausgewählt ist.

16. Verfahren nach einem der Ansprüche 12 bis 15, **dadurch gekennzeichnet, dass** die Temperatur T₂ der Siedetemperatur des Lösemittels entspricht.

17. Verwendung von Salzen nach einem der Ansprüche 1 bis 5 als Elektrolyt für eine Batterie.

18. Elektrolytzusammensetzung, die neben den Salzen nach einem der Ansprüche 1 bis 5 mindestens ein Salz enthält, das unter LiPF6, LiBF4, CF3COOLi, CF3SO2Li, LiTFSI (Lithium-bis(trifluormethansulfonyl)imid), LiFSI (Lithium-bis(fluorsulfonyl)imid), LiTDI (Lithium-1-trifluormethyl-4,5-dicarbonitril-imidazolat) und LiPDI (Lithium-1-pentafluorethyl-4,5-dicarbonitril-imidazolat) ausgewählt ist.

19. Verwendung nach Anspruch 17, **dadurch gekennzeichnet, dass** die Salze in einem Lösemittel solubilisiert sind, das vorzugsweise unter Carbonaten, Nitrilen und Glyme ausgewählt ist.

## Claims

1. A salt of the bicyclic imidazole compound (IV) represented by the expanded general formulae below: 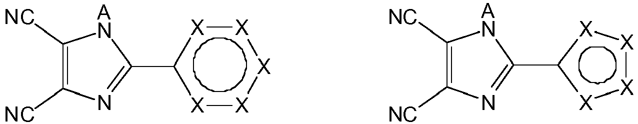 in which A represents a monovalent cation and X independently represents a carbon atom, an oxygen atom, a sulfur atom, a phosphorus atom or a nitrogen atom.

2. The salt as claimed in claim 1, **characterized in that** the monovalent cation A is an alkali metal, preferably chosen from lithium or sodium.

3. The salt as claimed in claim 1 or 2, **characterized in that** X represents a carbon, phosphorus or nitrogen atom.

4. The salt as claimed in claim 3, **characterized in that** said salt is substituted with an electron-withdrawing or an electron-donating group chosen from a cyano (CN) group, an R₁ group, an ether group of OR₁ type, an amino group of N(R₁)₂ type, an ester group of CO₂R₁ type, a sulfonyl group of SO₂R₁ type or a phosphonyl group of PO₂R₁ type, where R₁ has the formula CₙHₘX'ₚ with n between 0 and 6, m between 0 and 13, X' a halogen (F, Cl, Br and I) and p between 1 and 13.

5. The salt as claimed in claim 4, **characterized in that** the electron-withdrawing or electron-donating group is chosen from hydrogen, fluorine, the cyano (CN) group, the trifluoromethyl (CF₃) group, the trifluoromethoxy (OCF₃) group or the methoxy (OCH₃) group.

6. A process for the preparation of a salt as claimed in any one of claims 1 to 5, **characterized in that** an imidazole compound (III) represented by the expanded general formulae below: 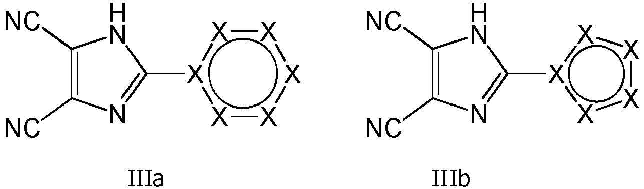 is reacted with a base AZ, with A having the same meaning as above and Z representing a hydride, hydroxide or carbonate anion.

7. The process as claimed in claim 6, **characterized in that** the base AZ is chosen from lithium hydride, lithium carbonate, lithium hydroxide, sodium hydride, sodium carbonate, sodium hydroxide and the combinations of these.

8. The process as claimed in claim 6 or 7, **characterized in that** the imidazole compound (III) is obtained by reacting diaminomaleonitrile with an aromatic cyclic acid derivative of formula (II) represented by the expanded general formulae below:

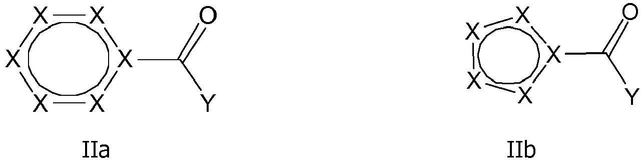

9. The process as claimed in claim 8, comprising (i) a stage of reaction of diaminomaleonitrile with an aromatic cyclic acid derivative of formula (II) at a temperature T₁ of between 0 and 80°C, preferably from 10 to 50°C, more preferably from 20 to 30°C, optionally in the presence of a solvent, to give a compound of formula (V) represented by the expanded general formulae below: 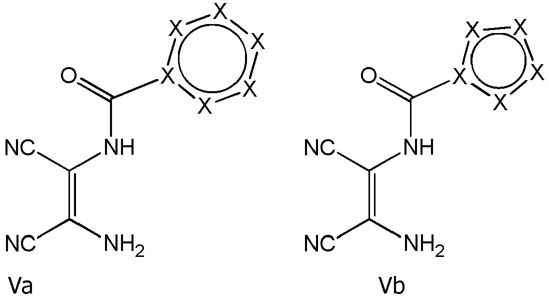 followed (ii) by a stage during which the compound of formula (V) is subjected to a heat treatment at a temperature T₂ with T₂ > T₁.

10. The process as claimed in claim 9, **characterized in that** the temperature T₂ is greater than T₁ by at least 10°C, or by at least 20°C, or by at least 30°C, or by at least 40°C, or by at least 50°C, or by at least 60°C, or by at least 70°C.

11. The process as claimed in claim 9 or 10, **characterized in that** stage (ii) is carried out immediately following the first stage without intermediate purification.

12. The process as claimed in any one of claims 8 to 11, **characterized in that** stage (i) is carried out in the presence of a solvent.

13. The process as claimed in claim 12, **characterized in that** the solvent is chosen from dioxane, toluene, acetonitrile or dimethylformamide.

14. The process as claimed in any one of claims 8 to 13, **characterized in that** stage (ii) is carried out in the presence of an acid catalyst.

15. The process as claimed in claim 14, **characterized in that** the acid catalyst is chosen from trifluoroacetic acid, acetic acid or benzoic acid.

16. The process as claimed in any one of claims 12 to 16, **characterized in that** the temperature T₂ corresponds to the boiling point of the solvent.

17. The use of the salt as claimed in any one of claims 1 to 5 as electrolyte component for a battery.

18. An electrolyte composition comprising, in addition to the salt as claimed in any one of claims 1 to 5, at least one salt chosen from LiPF₆, LiBF₄, CF₃COOLi, CF₃SO₂Li, LiTFSI (lithium bis(trifluoromethanesulfonyl)imide), LiFSI (lithium bis(fluorosulfonyl)imide), LiTDI (lithium 4,5-dicyano-2-(trifluoromethyl)imidazolide) and LiPDI (lithium 4,5-dicyano-2-(pentafluoroethyl)imidazolide).

19. The use as claimed in claim 17, **characterized in that** the salt is dissolved in a solvent, preferably chosen from carbonates, nitriles or glymes.
